(19) Europäisches Patentamt
European Patent Office
Office européen des brevets

(11) EP 3 311 884 A1

(12) EUROPEAN PATENT APPLICATION

(43) Date of publication:
25.04.2018 Bulletin 2018/17

(51) Int Cl.:
A61N 5/10 (2006.01)

(21) Application number: 16194392.3

(22) Date of filing: 18.10.2016

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: SUMITOMO HEAVY INDUSTRIES, LTD.
Tokyo 141-6025 (JP)

(72) Inventor: MUKAWA, Tetsuya
Yokosuka-shi,, Kanagawa 237-8555 (JP)

(74) Representative: Walcher, Armin
Louis, Pöhlau, Lohrentz
Patentanwälte
Postfach 30 55
90014 Nürnberg (DE)

(54) RADIATION TREATMENT SIMULATION APPARATUS

(57) An object of the invention aims is to provide a radiation treatment simulation apparatus (10) that improves the simulation precision of a therapeutic dose in radiation treatment. The radiation treatment simulation apparatus (10) includes a calculating unit (5) that calculates the therapeutic dose distribution of radiation with which a patient is to be irradiated in the radiation treatment, and an information storage unit (3) that stores information on an administered medicine that is a sensitizer or a protective agent to be administered to a patient in the case of the radiation treatment, and a calculating unit (5) calculates the therapeutic dose distribution on the basis of the information on the administered medicine stored in the information storage unit (3).

FIG. 1

EP 3 311 884 A1

**Description**

BACKGROUND OF THE INVENTION

Field of the Invention

A certain embodiment of the present invention relates to a radiation treatment simulation apparatus.

Description of Related Art

In the related art, for example, a particle beam treatment program apparatus described in the following Japanese Unexamined Patent Application Publication No. 2011-217902 is known as a technique of such a field. This apparatus includes target region input means for inputting a target region serving as a target to be irradiated with a particle beam, and a calculating device that determines an irradiation place to be irradiated with the particle beam within an irradiation region set so as to include the target region. This calculating device determines the irradiation place such that the irradiation place is disposed on the contour of the irradiation region, and the spacing between irradiation spots adjacent to each other is equal or smaller than a predetermined set value.

SUMMARY OF THE INVENTION

In this type of treatment program apparatus, improvement in treatment program precision is required in order to reduce a radiation dose to be given to a patient's normal tissue and improve a radiation dose to tumor tissue. In order to solve this problem, an object of the invention aims is to provide a radiation treatment simulation apparatus that improves the simulation precision of a therapeutic dose in radiation treatment.

Aradiation treatment simulation apparatus of the invention includes a therapeutic dose distribution calculator that calculates a therapeutic dose distribution of radiation with which a patient is to be irradiated in radiation treatment; and a medicine information storage unit that stores information on an administered medicine that is a sensitizer or a protective agent to be administered to the patient in the case of the radiation treatment. The therapeutic dose distribution calculator calculates the therapeutic dose distribution on the basis of information on the administered medicine stored in the medicine information storage unit.

According to this apparatus, a high-precision therapeutic dose distribution including the influence of the sensitizer or protective agent to be administered to a patient can be obtained.

Additionally, when calculating a therapeutic dose in a minute region within an irradiation region of the radiation, the therapeutic dose distribution calculator may calculate the therapeutic dose of the minute region on the basis of unit concentration variation ratio information showing an increase rate of the influence of the radiation per unit concentration of the administered medicine in the minute region, and administered medicine concentration information showing the concentration of the administered medicine that is present in the minute region.

Additionally, the therapeutic dose distribution calculator may calculate the therapeutic dose distribution in a case where the administered medicine is administered to the patient, and the therapeutic dose distribution in a case where the administered medicine is not administered to the patient, and an information display unit may be further included to display the therapeutic dose distribution in a case where the administered medicine is administered to the patient and the therapeutic dose distribution in a case where the administered medicine is not administered to the patient, on the basis of results calculated in the therapeutic dose distribution calculator. According to this configuration, a user can ascertain the influence of the administered medicine visually.

Additionally, specifically, the above radiation may be a charged particle beam, X-rays, a gamma beam, or an electron beam. Advantageous Effects of Invention

According to the radiation treatment simulation apparatus of the invention, the simulation precision of a therapeutic dose in radiation treatment can be improved.

BRIEF DESCRIPTION OF THE DRAWINGS

FIG. 1 is a block diagram illustrating an embodiment of a radiation treatment simulation apparatus.
FIG. 2 is a block diagram illustrating a hardware configuration of the radiation treatment simulation apparatus.
FIG. 3 is a schematic view illustrating an embodiment of a charged particle beam irradiation apparatus.

DETAILED DESCRIPTION OF THE INVENTION

Hereinafter, an embodiment of a radiation treatment simulation apparatus related to the invention will be described in detail, referring to the drawings.

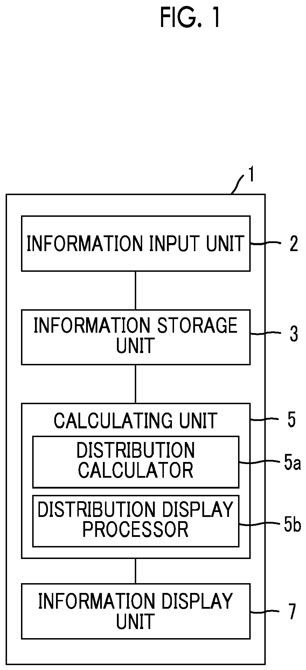

A radiation treatment simulation apparatus 10 of the present embodiment illustrated in FIG. 1 calculates therapeutic dose distribution of radiation with which a patient is to be irradiated, in radiation treatment using a radiation treatment apparatus. A charged particle beam, X-rays, a gamma beam, or an electron beam, for example, is included in the above radiation targeted by the radiation treatment simulation apparatus 10. Additionally, a proton ray beam and a heavy particle beam are included in the above charged particle beam.

FIG. 1 is a block diagram illustrating functional elements of the radiation treatment simulation apparatus 10 in blocks. The radiation treatment simulation apparatus 10 has an information input unit 2, an information storage unit 3 (medicine information storage unit), a calculating unit 5 (therapeutic dose distribution calculator), and an information display unit 7 (information display unit).

The information input unit 2 receives information input from a user for each simulation apparatus. The information storage unit 3 stores information required for simulation calculation temporarily or for a long period of time. As information stored in the information storage unit 3, for example, there is input information that a user input from the information input unit 2. Additionally, predetermined constants or the like required for simulation calculation are determined in advance and stored in the information storage unit 3.

The calculating unit 5 has a distribution calculator 5a and a distribution display processor 5b. The distribution calculator 5a calculates therapeutic dose distribution in a radiation-irradiated region with a predetermined algorithm, on the basis of information imparted from the information storage unit 3. Specifically, the distribution calculator 5a splits the irradiation region into minute region, and calculates the therapeutic doses of the individual minute regions, respectively. Then, the therapeutic dose distribution of the irradiation region is obtained as a set of the therapeutic doses of the individual minute regions. The distribution display processor 5b executes, for example, image processing based on the therapeutic dose distribution obtained by the distribution calculator 5a, and draws, for example, a contour diagram illustrating the therapeutic dose distribution. The information display unit 7 displays the therapeutic dose distribution of the irradiation region as a calculation result of the calculating unit 5 on a screen. For example, the information display unit 7 is constituted with a display device of a computer.

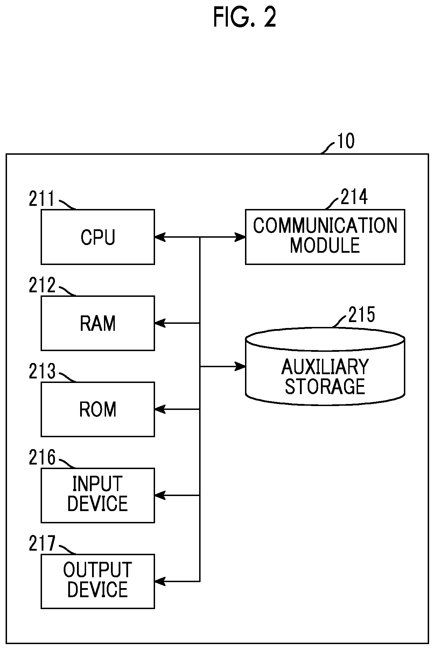

Here, an example of a physical configuration of the radiation treatment simulation apparatus 10 will be described. FIG. 2 is a hardware configuration view of the radiation treatment simulation apparatus 10. As illustrated in the drawing, the radiation treatment simulation apparatus 10 is physically constituted as a computer system including a CPU 211, a RAM 212 and a ROM 213 that are main storages, an auxiliary storage 215, such as a hard disk, an input device 216, such as a keyboard and a mouse that are input devices, an output device 217, such as a display, a communication module 214 that is a data transmission-and-reception device, such as a network card, and the like. The respective functions described referring to FIG. 1 is executed by making predetermined computer software read on hardware, such as the CPU 211 and the RAM 212 illustrated in FIG. 2, thereby operating the communication module 214, the input device 216, and the output device 217 under control of the CPU 211 and performing reading and writing of data in the RAM 212 or the auxiliary storage 215.

Meanwhile, in radiation treatment, a sensitizer or a protective agent may be administered to a patient in the case of treatment. For example, there is a problem that tumor tissue that falls into a hypoxic state has strong radioresistance and a radiation irradiation effect is low in such tumor tissue. As a countermeasure against this, medicine or the like for releasing the hypoxic state is administered to a patient. The medicine or the like for releasing such a hypoxic state is included as an example of the above sensitizer. Additionally, medicine that reduces the DNA chain cutting effect resulting from radiation is included as an example of the above protective agent. This medicine is administered to a patient's normal tissue so as to reduce any damage to the normal tissue, thereby allowing an increase in the amount of radiation dose to a tumor. This kind of a sensitizer or a protective agent influences the therapeutic dose as can be understood from the intended use thereof. Therefore, if the influence resulting from the sensitizer or the protective agent to be administered to a patient is included in consideration when calculating the therapeutic dose, higher-precision therapeutic dose distribution can be obtained.

In view of this knowledge, in the radiation treatment simulation apparatus 10, the therapeutic dose distribution including information on the sensitizer or the protective agent (hereinafter referred to as an "administered medicine") to be administered to a patient at the time of treatment in consideration is obtained.

Hereinafter, specific calculation processing using the radiation treatment simulation apparatus 10 will be described.

In the radiation treatment simulation apparatus 10, required information is input by a user for each simulation processing, and is stored in the information storage unit 3. Additionally, constants or the like that does not fluctuate for each simulation processing is stored in advance in the information storage unit 3.

As described above, the distribution calculator 5a of the calculating unit 5 splits the radiation-irradiated region into the minute regions, and calculates the therapeutic doses of the individual minute regions, respectively, on the basis of the information stored in the information storage unit 3. Then, the therapeutic dose distribution of the irradiation region is obtained as a set of the therapeutic doses of the individual minute regions. In this case, in the distribution calculator 5a, a therapeutic dose D [Gy-eq] in one minute region within the radiation irradiation region is calculated by a calculation algorithm expressed by the following Expression (1).

$$[\text{Expression 1}]$$

$$D = T \times \sum_{\gamma=1}^{i} \sum_{c=0}^{j} \{f_\gamma \times \varphi_\gamma \times RBE_\gamma \times (CF_{\gamma,c} \times \rho_c)\}$$

Here,

D: Therapeutic dose [Gy-eq]
T: Irradiation time [s]
$f_r$: Radiation dose conversion factor of radiation type r [Gy/(n/cm$^2$)]
$\phi_r$: Ray flux of radiation type r [n/cm$^2$/s]
$RBE_r$: Radiobiological effect ratio of radiation type r [Gy-eq/Gy]
$CF_{r,c}$: Variation ratio to radiation type r per unit concentration of administered medicine c (where, in the case of c = 0 (with no medicine), regarded as CF = 1 and no influence)
$\rho_c$: Concentration of administered medicine c (where, in the case of c= 0 (with no medicine), regarded as p = 1 and no influence)
r: Type of radiation
c: Type of administered medicine

For example, in a case where two types (for example, a proton beam r1 and a gamma beam r2) of radiations to be taken into consideration in calculation of the therapeutic dose D are present, and two types (referred to as c1 and c2) of administered medicines to be administered to a patient and be taken into consideration are present, Expression (1) can be written as follows.

$$D = T \times (f_{r1} \times \phi_{r1} \times RBE_{r1}$$
$$+ f_{r1} \times \phi_{r1} \times RBE_{r1} \times CF_{r1,c1} \times \rho_{c1}$$
$$+ f_{r1} \times \phi_{r1} \times RBE_{r1} \times CF_{r1,c2} \times \rho_{c2}$$
$$+ f_{r2} \times \phi_{r2} \times RBE_{r2}$$
$$+ f_{r2} \times \phi_{r2} \times RBE_{r2} \times CF_{r2,c1} \times \rho_{c1}$$
$$+ f_{r2} \times \phi_{r2} \times RBE_{r2} \times CF_{r2,c2} \times \rho_{c2}) \qquad (1)'$$

Some (for example, irradiation time T) of individual parameters to be input to the above Expression (1) are input from a user to the radiation treatment simulation apparatus 10 for each simulation processing, and are stored in the information storage unit 3. Additionally, other some of the individual parameters to be input to the above Expression (1) are calculated by the distribution calculator 5a on the basis of the information input from a user and stored in the information storage unit 3, as described above. For example, the concentration $\rho_c$ of an administered medicine is calculated according to a well-known operational expression by the distribution calculator 5a on the basis of the information (for example, the amount of the administered medicine administered to a patient or the like) input from the user to the radiation treatment simulation apparatus 10 for each simulation processing.

Additionally, further other some of the respective parameters input to the above Expression (1) are stored in advance in the information storage unit 3 as constants. For example, the value of $CF_{r,c}$ that is information on the administered medicine is determined for each combination of the type of the administered medicine and the type of radiation, is stored in the information storage unit 3, and is handed over to the distribution calculator 5a at the time of calculation. In addition, $CF_{r,c}$ (unit concentration variation ratio information) showing the increase rate of the influence of a certain radiation r on a minute region to be observed per the unit concentration (1 ppm) of a certain administered medicine c. Additionally, $\rho_c$ (administered medicine concentration information) shows the concentration of the certain administered medicine c that is present in the minute region to be observed.

The distribution calculator 5a executes the above calculation on all the minute regions within the irradiation region, and calculates therapeutic doses D corresponding to all the minute regions. Then, in the distribution calculator 5a, therapeutic dose distribution (hereinafter referred to as a "first therapeutic dose distribution") of the overall irradiation region is obtained as a set of the therapeutic doses D of the individual minute regions.

Next, the distribution calculator 5a calculates the therapeutic dose distribution (hereinafter referred to as a "second therapeutic dose distribution") of the overall irradiation region in a case where it is assumed that an administered medicine is not administered to a patient under the same irradiation conditions as above. As a calculation technique of calculating the second therapeutic dose distribution, for example, $CF_{r,c} \times \rho_c = 1$ may be established in Expression (1).

The distribution calculator 5a hands over the first therapeutic dose distribution and the second therapeutic dose distribution to the distribution display processor 5b. Then, the distribution display processor 5b converts the first therapeutic dose distribution and the second therapeutic dose distribution into, for example, image information, such as contour diagrams. This image information is handed over to the information display unit 7, and for example, a contour diagram of the first therapeutic dose distribution and a contour diagram of the second therapeutic dose distribution are displayed in parallel on a screen.

Subsequently, working effects exhibited by the radiation treatment simulation apparatus 10 will be described. With respect to the aforementioned Expression (1), an example of a related-art calculation algorithm for therapeutic dose that is generally known is expressed by the following Expression (2).

[Expression 2]

$$D = T \times \sum_{\gamma=1}^{i} (f_\gamma \times \varphi_\gamma \times RBE_\gamma)$$

In Expression (1) to be used in the radiation treatment simulation apparatus 10, the therapeutic dose distribution based on the information on the administeredmedicine to be administered to a patient at the time of treatment ($CF_{r,c}$, and $\rho_c$) is obtained as compared to Expression (2). That is, in Expression (1), an item ($CF_{r,c} \times \rho_c$) relevant to the administered medicine is added as compared to Expression (2). Hereinafter, the item ($CF_{r,c} \times \rho_c$) is referred to as a medicine-related item.

The influence of the administered medicine is quantitatively reflected in the therapeutic dose D due to the presence of the above medicine-related item in Expression (1). Therefore, according to the radiation treatment simulation apparatus 10 adopting the calculation algorithm of Expression (1), a high-precision therapeutic dose distribution including the influence of the sensitizer or the protective agent to be administered to a patient can be obtained. As a result, the simulation precision of the therapeutic dose in radiation treatment can be improved.

Additionally, since a therapeutic dose distribution (first therapeutic dose distribution) in a case where an administered medicine is administered to a patient and a therapeutic dose distribution (second therapeutic dose distribution) in a case where the administered medicine is not administered to the patient are displayed by the information display unit 7, a user can visually ascertain the influence of the administered medicine by comparing both of the therapeutic dose distributions.

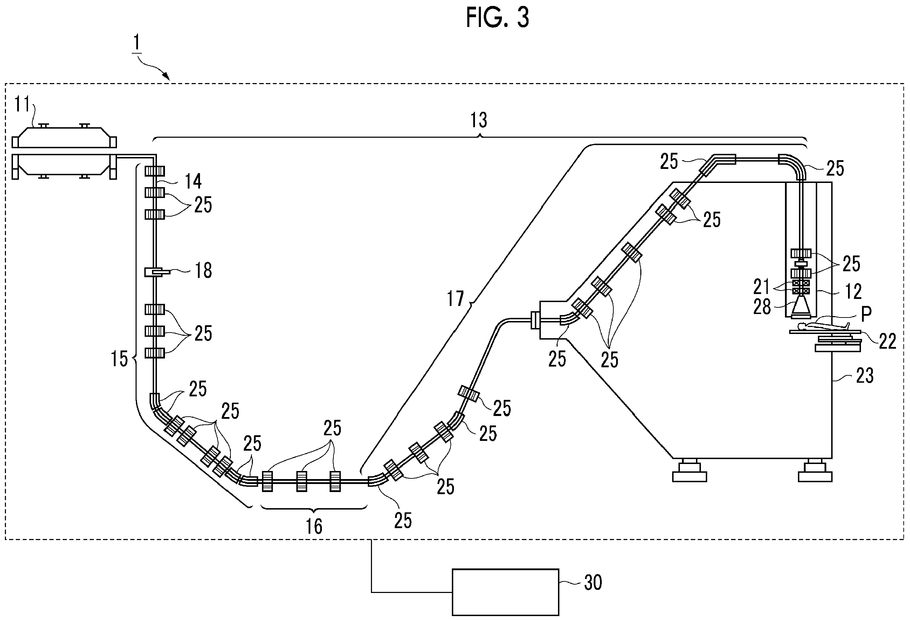

The radiation treatment simulation apparatus 10 of the above-described present embodiment can be used for, for example, calculation of the therapeutic dose distribution of a charged particle beam irradiation region in a charged particle beam treatment apparatus to be described below, and can be utilized for a treatment program. Hereinafter, the charged particle beam treatment apparatus 1 will be described in detail, referring to FIG. 3. In addition, the terms "upstream" and "downstream" mean an upstream (accelerator side) and a downstream (patient side) of a charged particle beam to be emitted, respectively.

As illustrated in FIG. 3, the charged particle beam treatment apparatus 1 includes an accelerator 11 that is a device used for cancer treatment or the like by radiotherapy and accelerates charged particles to emit a charged particle beam, an irradiation nozzle 12 (irradiation unit) that irradiates a body to be irradiated with a charged particle beam, a beam transportation line 13 (transportation line) that transports the charged particle beam emitted from the accelerator 11 to the irradiation nozzle 12, a degrader (energy adjusting unit) 18 that is provided in the beam transportation line 13 to lower the energy of the charged particle beam to adjust the range of the charged particle beam, a plurality of electromagnets 25 that are provided in the beam transportation line 13, and a control unit 30 that controls the overall charged particle beam treatment apparatus 1. In the present embodiment, although a cyclotron is adopted as the accelerator 11, the accelerator is not limited to this, and may be other generation sources, for example, a synchrotron, synchrocyclotron, a linac, or the like that generate a charged particle beam.

In the charged particle beam treatment apparatus 1, irradiation of a charged particle beam emitted from the accelerator 11 to a tumor (body to be irradiated) of the patient P on a treatment table 22 is performed. The charged particle beam is obtained by accelerating particles at high speed with electric charges, and includes, for example, has a proton beam, a heavy particle (heavy ion) beam, or the like.

The irradiation nozzle 12 is attached to the inside of a rotating gantry 23 so as to be rotatable 360° around the treatment table 22 and is made to be movable at arbitrary rotational positions by the rotating gantry 23. The electromagnets 25, scanning electromagnets 21, and a vacuum duct 28 are included in the irradiation nozzle 12. The scanning electromagnets 21 are provided in the irradiation nozzle 12. The scanning electromagnet 21 has an X-direction scanning electromagnet that scans a charged particle beam in an X direction in a plane intersecting an irradiation direction of the charged particle beam, and a Y-direction scanning electromagnet that scans a charged particle beam in a Y direction intersecting the X direction in a plane intersecting the irradiation direction of the charged particle beam. Additionally, since the charged particle beam scanned by the scanning electromagnet 21 is deflected in the X direction and/or a Y direction, the vacuum duct 28 on the downstream side has an increased diameter toward the downstream side than the scanning electromagnet.

The beam transportation line 13 has a vacuum duct 14 through which a charged particle beam passes. The inside of the vacuum duct 14 is maintained in vacuum, and suppresses that charged particles that constitute a charged particle beam under transportation are scattered due to air or the like.

Additionally, the beam transportation line 13 has an energy selection system (ESS) 15 that selectively takes out, from a charged particle beam with a predetermined energy width emitted from the accelerator 11, a charged particle beam with an energy width narrower than the predetermined energy width, a beam transport system (BTS) 16 that transports the charged particle beam with the energy width selected by the ESS 15 in a state where energy is maintained, and a gantry transport system (GTS) 17 that transports the charged particle beam from the BTS 16 toward the rotating gantry 23.

The degrader 18 lowers the energy of a charged particle beam to pass therethrough, and adjusts the range of the charged particle beam. Since the depth from a patient's body surface to a tumor that is a body to be irradiated varies for each patient, it is necessary to adjust a range that is an arrival depth of the charged particle beam when a patient is irradiated with a charged particle beam. The degrader 18 adjusts the energy of a charged particle beam emitted with constant energy from the accelerator 11, thereby adjusting the charged particle beam such that the charged particle beam appropriately reaches the body to be irradiated at a predetermined depth inside a patient's body. Energy adjustment of a charged particle beam by such degrader 18 is performed for each layer obtained by slicing the body to be irradiated.

The invention can be implemented in various forms in which various changes and improvements are made to the above-described embodiment on the basis of the knowledge of a person's skilled in the art. Additionally, it is also possible to configure modification examples of examples using the technical matter described in the above-described embodiment. The configurations of respective embodiments may be appropriately combined together.

**Claims**

1. A radiation treatment simulation apparatus (10) comprising:

   a therapeutic dose distribution calculator (5) that calculates a therapeutic dose distribution of radiation with which a patient is to be irradiated in radiation treatment; and
   a medicine information storage unit (3) that stores information on an administered medicine that is a sensitizer or a protective agent to be administered to the patient in the case of the radiation treatment,

   wherein the therapeutic dose distribution calculator (5) calculates the therapeutic dose distribution on the basis of information on the administered medicine stored in the medicine information storage unit (3).

2. The radiation treatment simulation apparatus according to Claim 1,
   wherein when calculating a therapeutic dose in a minute region within an irradiation region of the radiation, the therapeutic dose distribution calculator (5) calculates the therapeutic dose of the minute region on the basis of unit concentration variation ratio information showing an increase rate of the influence of the radiation per unit concentration of the administered medicine in the minute region, and administered medicine concentration information showing the concentration of the administered medicine that is present in the minute region.

3. The radiation treatment simulation apparatus according to Claim 1 or 2,
   wherein the therapeutic dose distribution calculator (5) calculates the therapeutic dose distribution in a case where the administered medicine is administered to the patient, and the therapeutic dose distribution in a case where the administered medicine is not administered to the patient, and
   wherein an information display unit (7 ) is further included to display the therapeutic dose distribution in a case where the administered medicine is administered to the patient and the therapeutic dose distribution in a case where the administered medicine is not administered to the patient, on the basis of results calculated in the therapeutic dose distribution calculator.

4. The radiation treatment simulation apparatus according to any one of Claims 1 to 3,
   wherein the radiation is a charged particle beam, X-rays, a gamma beam, or an electron beam.

# FIG. 1

```
                                                    ┌─ 1
┌──────────────────────────────────────┐
│                                        │
│   ┌──────────────────────────────┐    │
│   │   INFORMATION INPUT UNIT      │────┼── 2
│   └──────────────────────────────┘    │
│                 │                      │
│   ┌──────────────────────────────┐    │
│   │   INFORMATION STORAGE         │────┼── 3
│   │   UNIT                        │    │
│   └──────────────────────────────┘    │
│                 │                      │
│   ┌──────────────────────────────┐    │
│   │   CALCULATING UNIT            │────┼── 5
│   │  ┌────────────────────────┐   │    │
│   │  │  DISTRIBUTION          │───┼────┼── 5a
│   │  │  CALCULATOR            │   │    │
│   │  └────────────────────────┘   │    │
│   │  ┌────────────────────────┐   │    │
│   │  │  DISTRIBUTION DISPLAY  │───┼────┼── 5b
│   │  │  PROCESSOR             │   │    │
│   │  └────────────────────────┘   │    │
│   └──────────────────────────────┘    │
│                 │                      │
│   ┌──────────────────────────────┐    │
│   │   INFORMATION DISPLAY         │────┼── 7
│   │   UNIT                        │    │
│   └──────────────────────────────┘    │
│                                        │
└──────────────────────────────────────┘
```

FIG. 2

FIG. 3

**EUROPEAN SEARCH REPORT**

Application Number

EP 16 19 4392

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/206611 A1 (COLLINS WILLIAM F [US]) 6 November 2003 (2003-11-06) * paragraph [0009] * * paragraph [0010] * * paragraph [0071]; claims 5,12,15,21,22,24; figures 1,3 * * paragraph [0074] * * paragraph [0011] * | 1-4 | INV. A61N5/10 |
| X | US 2005/276377 A1 (CAROL MARK P [US]) 15 December 2005 (2005-12-15) * paragraph [0042] * * paragraph [0044] * * paragraph [0051] * * paragraph [0052] * * paragraph [0128] * * paragraph [0130] * * paragraph [0132] * * paragraph [0155] * * paragraph [0159] * * paragraph [0164] * * paragraph [0175] * * paragraph [0178] * | 1-4 | |
| X | US 2006/039533 A1 (WEIL MICHAEL D [US] ET AL) 23 February 2006 (2006-02-23) * paragraph [0041]; figures 1-4 * * paragraph [0044] * * paragraph [0045] * | 1-4 | TECHNICAL FIELDS SEARCHED (IPC)  A61N |
| A | US 2007/031337 A1 (SCHULTE REINHARD [US]) 8 February 2007 (2007-02-08) * claims 13,14 * | 4 | |

The present search report has been drawn up for all claims

| Place of search | Date of completion of the search | Examiner |
|---|---|---|
| Munich | 14 February 2017 | Rodríguez Cosío, J |

EPO FORM 1503 03.82 (P04C01)

## ANNEX TO THE EUROPEAN SEARCH REPORT
## ON EUROPEAN PATENT APPLICATION NO.

EP 16 19 4392

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2017

| Patent document cited in search report | | Publication date | Patent family member(s) | | Publication date |
|---|---|---|---|---|---|
| US 2003206611 | A1 | 06-11-2003 | AU | 2003270849 A1 | 08-04-2004 |
| | | | US | 2003206611 A1 | 06-11-2003 |
| | | | WO | 2004026403 A1 | 01-04-2004 |
| US 2005276377 | A1 | 15-12-2005 | US | 2005276377 A1 | 15-12-2005 |
| | | | US | 2009154646 A1 | 18-06-2009 |
| | | | US | 2012093293 A1 | 19-04-2012 |
| | | | US | 2014205067 A1 | 24-07-2014 |
| US 2006039533 | A1 | 23-02-2006 | EP | 1541196 A1 | 15-06-2005 |
| | | | JP | 2005169127 A | 30-06-2005 |
| | | | US | 2006039533 A1 | 23-02-2006 |
| US 2007031337 | A1 | 08-02-2007 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011217902 A